# EUROPEAN PATENT APPLICATION

(11) **EP 1 721 889 A1**
(43) Date of publication of application: **15.11.2006**
(21) Application number: 05103988.1
(22) Date of filing: 12.05.2005
(51) Int. Cl.: C07C 213/02, C07C 215/42, C07C 217/24, A61K 31/135

(54) **Process for the preparation of phenethylamine derivatives**

(71) Applicant: Dishman Pharmaceuticals & Chemicals Ltd., Navrangpura, 380 009 Ahmedabad (IN)
(72) Inventor: Rajnikant Vyas, Janmejay, W1H 5LP, London (GB); Satya Varma Nidadavolu, Venkata, 380 058, Ahmedabad (IN)
(74) Representative: van Westenbrugge, Andries

(57) **Abstract**

The present invention relates to a process of preparing venlafaxine and derivatives thereof comprising reducing the corresponding β-hydroxynitriles using a reducing borohydride agent selected from tetraalkylammonium borohydride and aralkyltrialkylammonium borohydride. According to the present process, said β-hydroxynitrile can be prepared by condensating a phenylacetonitrile and cyclohexanone using a phase transfer catalyst and a base at a temperature of between 10-25 °C. Advantageously, in the present method, the use of hazardous catalysts and reducing agents is avoided and unexpectedly low amounts of reducing borohydride agent can be used.

## Description

### Field of the invention

The present invention relates to a process of preparing phenethylamine derivatives, especially 1-(2-amino-1-[4-methoxyphenyl]ethyl)cyclohexanol, and salts thereof. Said phenethylamine derivatives are intermediates in the synthesis of certain pharmaceutically useful compounds, such as venlafaxine. The invention also relates to processes of preparing said pharmaceutically useful compounds.

### Background of the invention

The compound 1-(2-dimethylamino-1-[p-methoxyphenyl]ethyl)cyclohexanol, which is also referred to as venlafaxine, and pharmaceutically acceptable derivatives and salts thereof, have proven to be very useful central nervous system antidepressants, which act by inhibiting neuronal uptake of serotonin-norepinephrine and to a lesser extent dopamine.

Venlafaxine and several of its derivatives as well as their preparation are described in U.S. Patent No. 4,535,186. According to this document 1-(2-dimethylamino-1-[p-methoxyphenyl]ethyl)cyclohexanol is prepared by methylation of the intermediate 1-[2-amino-1-(p-methoxyphenyl)ethyl]cyclohexanol. This intermediate is produced by first coupling p-methoxyphenylacetonitrile and cyclohexanone using n-butyl lithium at a temperature of below -50 °C, and subsequently hydrogenating the obtained 1-[cyano(p-methoxyphenyl)methyl]cyclohexanol in the presence of a rhodium catalyst. According to US 4,535,186 the primary amine intermediate may also be obtained by reduction of the β-hydroxynitrile using borane reducing agents.

In U.S. Pat. No. 6,350,912 the process for the preparation of venlafaxine from the β-hydroxynitrile has been disclosed as a one pot process. According to said document venlafaxine is prepared by reduction of the corresponding β-hydroxynitrile in the presence of Raney nickel followed by conversion to venlafaxine with a formylating agent without prior isolation of the intermediate 1-[2-amino-1-(p-methoxyphenyl)ethyl]cyclohexanol. The β-hydroxynitrile is preferably prepared according to the process described in US 6,504,044. Said process comprises reacting cyclohexanone with aryl acetonitriles using a base and optionally a phase transfer catalyst, such as tetrabutylammonium iodide or tetrabutylammonium bromide.

WO 2002/50017 also relates to processes for preparing the intermediate 1-[2-amino-1-(p-methoxyphenyl)ethyl]cyclohexanol from the corresponding β-hydroxynitrile. According to this document the β-hydroxynitrile is hydrogenated in the presence of a nickel or cobalt catalyst.

US 2005/0033088 discloses the preparation of the same intermediate, 1-[2-amino-1-(4-methoxyphenyl)ethyl]cyclohexanol acetate, by reduction of 1-[cyano-(4-methoxyphenyl)methyl]cyclohexanol using palladium on carbon in the presence of organic acid at a pressure of between 5 and 25 kg/cm² and a temperature in the range of 30-75 °C. The intermediate compound is further converted to venlafaxine. The β-hydroxynitrile (1-[cyano-(4-methoxyphenyl)methyl]cyclohexanol) is produced by reacting 4-methoxybenzyl cyanide and cyclohexanone in a solution of sodium methoxide in methanol.

US 6,342,533 discloses the synthesis of optically pure venlafaxine derivatives. According to the method disclosed in said document, the β-hydroxynitrile is prepared by reacting cyclohexanone with 4-methoxyphenylacetonitrile in the presence of the catalyst lithium diisopropylamide (LDA), in an aprotic solvent such as THF. The cyano group of the compound so obtained is subsequently reacted with the reductant CoCl₂/NaBH₄ in methanol. According to the method disclosed in US 6,342,533, the sodium borohydride is applied in large excess, i.e. a ratio of approximately 10 moles per mole of 1-[cyano(4-methoxyphenyl)methyl]cyclohexanol.

From what is discussed here above, it may be clear that the processes described in the prior art each suffer from one or more drawbacks, e.g. relating to the catalysts that are used which are costly and some of them even hazardous, to the reaction conditions that have to be applied, e.g. hydrogen pressure or extremely low temperatures, and/or to the purity of the products/intermediates obtained, such that elaborate work up or purification processes are required, making these processes less convenient/attractive for large-scale preparation of venlafaxine and derivatives thereof.

Therefore it is the objective of the present invention to provide an improved process of preparing venlafaxine and derivatives thereof, and more in particular to provide an improved process of preparing the phenethylamine intermediate, wherein said intermediate is obtained in high purity and which process meets certain economical demands.

### Summary of the invention

The present inventors surprisingly found that phenethylamines, which are useful intermediates in the synthesis of venlafaxine and derivatives thereof, can suitably be produced by reducing the corresponding β-hydroxynitriles using a reducing borohydride agent selected from tetraalkylammonium borohydride and aralkyltrialkylammonium borohydride. More particularly, it was found that according to the present invention, the reduction can be performed in the presence of only a small excess of borohydride ions, which makes the present process more attractive than the prior art methods. Said reduction reaction can easily be performed at temperatures of between 0 and 30 °C, under atmospheric pressure.

The present inventors furthermore found that a particularly advantageous process is provided if the β-hydroxynitrile is prepared by condensating the phenylacetonitrile and cyclohexanone using a phase transfer catalyst and a base at a temperature of between 10-25 °C.

Hence, the present process provides the particular advantage that the use of hazardous catalysts and reducing agents is avoided. Moreover, the present inventors found that the relative amount of the reducing borohydride agent was unexpectedly low, i.e. the relative amount of tetraalkylammonium borohydride and/or aralkyltrialkylammonium borohydride, required is much smaller than that used in the prior art processes where sodium borohydride is used.

It is furthermore noted that the specific reaction conditions required for performing the respective reaction steps make the present process particularly attractive from an environmental and economic perspective..

In addition, the present process allows for the production of venlafaxine and derivatives thereof in such purity that no elaborate purification or work-up is required. More in particular, methylation of the phenethylamine derivatives prepared according to the present process directly yields venlafaxine or a derivative thereof without the need for further purification.

The synthetic pathway according to the present invention is depicted in the following scheme.

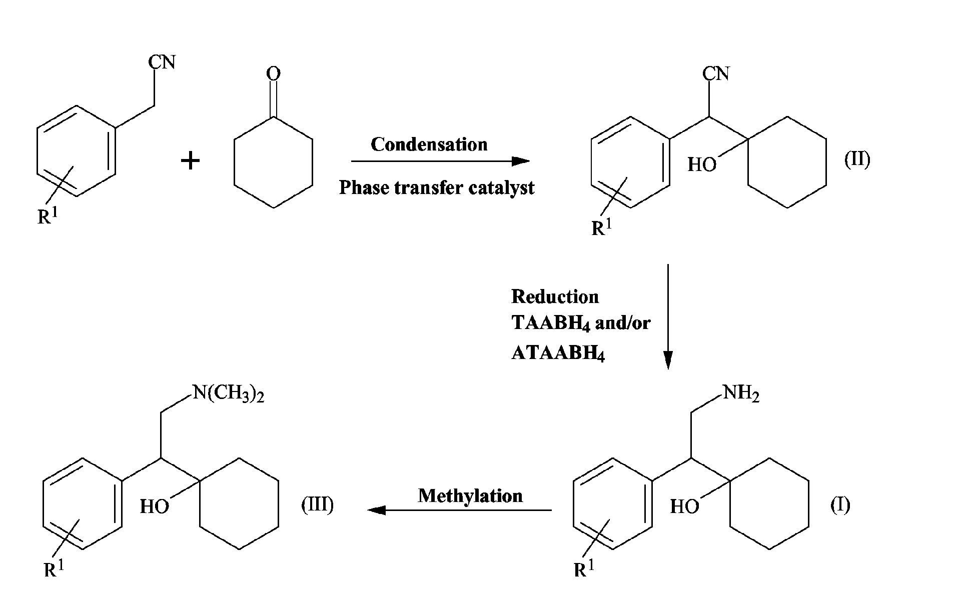

### Detailed description of the invention

Therefore, a first aspect of the present invention relates to a process for the preparation of a compound represented by formula (I) and salts thereof, wherein R¹ represents H, OH, or an optionally substituted alkyl or alkoxyl group; said process comprising reacting a compound represented by formula (II) and a reducing borohydride agent selected from tetraalkylammonium borohydride and aralkyltrialkylammonium borohydride in the presence of an alkylhalide.

As used herein the term "alkyl group" or "alkoxyl group" means straight chain or branched C₁-C₆ alkyl or C₁-C₆ alkoxyl, respectively, and "substituted" means substituted with one or more halogen, hydroxyl or alkoxyl groups.

Most preferably the present invention provides a process for the preparation of 1-(2-amino-1-[4-methoxyphenyl]ethyl)cyclohexanol, the phenethyl intermediate that is used to prepare venlafaxine. Hence, according to a preferred embodiment of the present process R¹ represents a para-methoxy group.

In this document, the term "the condensation" is used to refer to the reaction wherein the compound represented by formula (II) is prepared from cyclohexanone and a phenylacetonitrile as depicted in the above presented reaction scheme. Correspondingly, the term "the reduction" is used to refer to the reaction wherein the compound represented by formula (II) is converted to the compound represented by formula (I) and the term "the methylation" is used to refer to the conversion reaction of the compound represented by formula (I) to the compound represented by formula (III).

As mentioned herein before the reduction is performed in the presence of a reducing borohydride agent selected from tetraalkylammonium borohydride and aralkyltrialkylammonium borohydride, which allows for the reaction to be performed under mild conditions. It is preferred that the molar ratio of said reducing agent and the β-hydroxynitrile does not exceed 5:1, more preferably it does not exceed 3:1, most preferably it does not exceed 2:1. Said ratio preferably is at least 1:5. The present inventors found that by using a reducing borohydride agent selected from tetraalkylammonium borohydrides and aralkyltrialkylammonium borohydride instead of sodium borohydride, under similar conditions (especially temperature and pressure), the β-hydroxynitrile is reduced at approximately the same rate while using the borohydride in smaller excess.

According to the present invention the term 'tetraalkylammonium borohydride', as used herein, refers to the borohydride salts of quaternary alkylammonium compounds wherein the alkyl moieties are preferably selected from straight chain or branched C₁-C₆ alkyl moieties. The term 'aralkyltrialkylammonium borohydride' as used herein refers to the borohydride salts of quaternary aralkyltrialkylammonium compounds wherein the alkyl moieties are preferably selected from straight chain or branched C₁-C₆ alkyl moieties. The present reducing borohydride agent is preferably selected from the group of tetrabutylammonium borohydride, tetramethylammonium borohydride, tetraisoproplyammonium borohydride, tetrapropylammonium borohydride, tetraethylammonium borohydride, tetraisobutylammonium borohydride, tetra-tert-butylammonium borohydride, tetra-sec-butylammonium borohydride, benzyltrimethylammonium borohydride and benzyltriethylammonium borohydride. More preferably the tetraalkylammonium borohydride is selected from tetraethyl ammonium borohydride, tetramethylammonium borohydride, tetratpropylammonium borohydride and tetrabutyleammonium borohydride, most preferably tetrabutylammonium borohydride is used.

According to the present invention the term 'alkylhalide', as used herein, refers to alkyl compounds, preferably C₁₋₁₂ alkyl compounds, substituted with a halogen atom. According to a preferred embodiment the present alkylhalide is selected from methyliodide, ethyliodide, butyliodide and decylioide. Preferably the alkylhalide is selected from ethylioide and methyliodide, most preferably the alkylhalide is methyliodide.

The reduction is conveniently performed at a temperature of between 0 and 30 °C, preferably under atmospheric pressure, such that the reduction reaction of the β-hydroxynitrile is essentially completed within a period of between 0.5 and 4 hours, preferably between 1 and 3 hours, provided that the reducing borohydride agent selected from tetraalkylammonium borohydride and aralkyltrialkylammonium borohydride is applied in the aforementioned relative amount. It may be preferable to adjust the temperature when combining or adding the respective ingredients, as can be determined by the skilled person. Most preferably the temperature of the reaction mixture is maintained within the range of 25-30 °C once all reactants have been added and/or combined.

Preferably, in the present process, the reduction is performed in an organic solvent selected from hydrocarbons and halogenated hydrocarbons, such as pentane, hexane, toluene, dichloromethane, chlorobenzene and the like. Most preferably the organic solvent is selected from dichloromethane, chlorobenzene and toluene. Most preferably dichloromethane is used.

The pharmaceutically acceptable salts of the basic compounds of this invention are preferably acid addition salts, which are conveniently formed by reaction of the free base with an equivalent amount of any acid which forms a non toxic salt. Suitable examples of such acids include hydrochloric acid, hydrobromic acid, fumaric acid, maleic acid, succinic acid, sulphuric acid, phosphoric acid, tartaric acid, acetic acid, citric acid and the like. The formation of water soluble salts is preferred, in particular the hydrochloric acid addition salt.

Therefore, in a preferred embodiment, the present process further comprises passing hydrochloric acid gas through an organic solvent comprising the compound represented by formula (I) and isolating said compound as its hydrochloride salt by crystallization. Preferably hydrochloric acid gas is passed through said solvent comprising the phenethyl amine derivative for at least 15 minutes, more preferably at least 20 minutes. Preferably the temperature is maintained within the range of 25-30 °C while passing said hydrochloric acid gas through the solvent.

Furthermore, it is preferred that after the reduction step the compound according to formula (I) is extracted from the organic solvent with an aqueous solution and subsequently from the aqueous solution with a second organic solvent, which may be the same or a different kind of solvent as the one used in the reduction step.

Still more preferably, after the formation of the hydrochloride salt and crystallization thereof by removal of the organic solvent, the salt is recrystallized from acetone. This procedure allows for the obtained intermediate product to be used directly for the preparation of venlafaxine hydrochloride with such purity that it can be applied without further purification and/or work-up steps.

In a preferred embodiment of the present process, the condensation step, i.e. the preparation of the compound represented by formula (II), comprises reacting a phenylacetonitrile, preferably a phenylacetonitrile comprising a para or ortho hydroxyl, alkyl or alkoxyl group, and cyclohexanone in an aqueous hydroxide solution in the presence of a phase transfer catalyst.

According to a preferred embodiment said phase transfer catalyst is selected from tetraalkyl or aralkyltrialkylammonium salts, i.e. salts of quaternary alkylammonium compounds wherein the alkyl moieties are preferably selected from straight chain or branched C₁-C₆ alkyl moieties, such as tetrabutylammonium hydrogensulphate, tetrabutylammonium bromide, tetrabutylammonium chloride, tetrabutylammonium iodide and benzyltriethyl ammonium chloride. Most preferably the phase transfer catalyst is tetrabutylammonium bromide.

Preferably, the aqueous hydroxide solution is a sodium hydroxide solution or a potassium hydroxide solution, most preferably a sodium hydroxide solution. Preferably the aqueous hydroxide solution is a 5-50 wt% solution.

The condensation step is suitably performed at a temperature of between 10 and 25 °C, more preferably of between 15 and 18 °C, under atmospheric pressure. Under such conditions the condensation reaction is essentially completed within a period of between 0.5 and 4 hours, preferably of between 1 and 3 hours.

After performing the condensation reaction, the product, i.e. the compound represented by formula (II), may be isolated and/or purified by conventional methods such as washing and extraction and/or (re)crystallization. According to the present invention, elaborate work-up procedures such as chromatography are not required for obtaining a product of acceptable purity.

Another aspect of the present invention relates to a process for the preparation of a compound represented by formula (III), wherein R¹ represents H, OH, or an optionally substituted alkyl or alkoxyl group, and salts thereof comprising the step of preparing the compound represented by formula (I) and salts thereof, comprising combining in an organic solvent a compound represented by formula (II) and a reducing borohydride agent selected from tetrabutylammonium borohydride and aralkyltrialkylaamonium borohydride at a temperature of between 0-30 °C; and the subsequent step of methylating the compound represented by formula (I). Preferably the compound represented by formula (II) is prepared by reacting a phenylacetonitrile, preferably a phenylacetonitrile comprising a para or ortho hydroxyl-, alkyl- or alkoxyl group, and cyclohexanone in an aqueous hydroxide solution in the presence of a phase transfer catalyst at a temperature of between 10-25 °C.

According to a preferred embodiment the methylation comprises heating to reflux a combination of formaldehyde, formic acid, water and the compound represented by formula (I).

A still more preferred embodiment of the invention relates to a process for preparing venlafaxine and salts thereof is provided comprising the condensation step, the reduction step and the methylation step as defined herein before.

Yet another aspect of the present invention relates to a process for the preparation of a pharmaceutical composition containing 0.01-25 wt%, more preferably 0.05-10 wt%, of the compound represented by formula (III), said process comprising the step of preparing said compound represented by formula (III) according to the process as defined herein before and subsequently combining said compound represented by formula (III) with one or more pharmaceutically acceptable excipients. Preferably, said compound represented by formula (III) is venlafaxine hydrochloride and the one or more pharmaceutically acceptable excipients are those common for the preparation of orally ingestible formulations such as capsules or tablets or formulations for parenteral administration such as injectables.

### Examples

### Example 1: Preparation of 1-(cyano[4-methoxyphenyl]methylcyclohexanol

100 g 4-methoxyphenylacetonitrile (0.408 mole) was cooled to 15-18°C. 280 ml of an aqueous sodium hydroxide solution (0.68 mole in 280 ml of water) was added at 15-18 °C in 10 min. followed by 1 g of tetrabutylammonium bromide (TBAB). A first part, 45 g, of cyclohexanone (0.455 mole) was added to the above solution in 5 min followed by 0.4 g of tetrabutylammonium borohydride (TBAB). Subsequently, a second part, again 45 g, of cyclohexanone (0.455 mole) was added in 5 min. The temperature was maintained at 15-18 °C for 1.5 hrs. Completion of the reaction was confimed by thin layer chromatography (TLC). The reaction mass was filtered and washed with water till the mother liquor pH was neutral. The wet cake was dissolved in 300 ml of toluene and heated to 80-85 °C followed by cooling to 5-10 °C. The temperature was maintained at 5-10 °C for 30 min., followed by filtration and washing of the product with 100 ml of chilled toluene. The product was dried at 60-65 °C till the loss on drying (LOD) was below 0.5 % w/w, yield 144 g.

### Example 2: Preparation of 1-(2-amino-1-[4-methoxyphenyl]ethyl)cyclohexanol hydrochloride

25 g of 1-(cyano-[4-methoxy phenyl]methylcyclohexanol was charged in 205 ml of dichloromethane at 25 °C followed by 39.5 g of tetrabutylammonium borohydride, which was added in 10 min at 25 °C. The solution was cooled to 0-5°C followed by the slow addition of 29 g of methyl iodide in 30-45 min at 0-5 °C. The temperature was maintained at 0-5°C for 30 min., than slowly raised to 25-30 °C and maintained for 90 min at 25-30°C.
Completion of the reaction was checked by thin layer chromatography. 20 ml of ethanol was slowly added at 25-30 °C and stirred for 30 min. The pH was adjusted to 2.0 with hydrochloric acid (diluted) at 25-30 °C. The product was extracted from dichloromethane using 2 x 125 ml of demineralised water. The aqueous layer was collected and the pH therof was readjusted to 10.5 to 11.0 with 48 % sodium hydroxide solution. Subsequently the product was extracted from the aqueous layer with 2 x 100 ml dichloromethane, the dichloromethane was collected and dried over sodium sulfate. After removing the sodium sulfate by filtration, hydrochloric acid gas was passed through the dichloromethane layer for 15-20 min at 25-30 °C. The temperature was maintained at 25-30 °C for 30 min. The dichloromethane was removed completely under reduced pressure at a temperature below 45 °C, followed by the addition of 50 ml of acetone and cooling to 0-5 °C. After stirring for 60 min. at 0-5°C, the crystallized intermediate was filtered and washed with 25 ml of chilled (0-5 °C) acetone. After drying of the powder for 60 min at 55-60 °C the yield was 12.5 g.

### Example 3: Preparation of 1-(2-dimethylamino-1-[4-methoxyphenyl]ethyl)cyclohexanol hydrochloride

10 g of the product prepared in example-2, 11 ml of formaldehyde solution, 14.5 ml of formic acid and 111 ml of demineralised water were combined at room temperature. The mixture was heated to reflux and that temperature was maintained for 16 hrs (97-99°C). The mixture was than cooled to 30°C and acidified with 15% hydrochloric acid at 30°C. The aqueous layer was cooled to 10°C and the pH was adjusted to 10-11 using 48-50% sodium hydroxide solution. The aqueous layer was extracted with toluene, the toluene layer filtered over hi-flow and subsequently dried over sodium sulphate. The toluene was removed under vacuum below 65 °C, followed by the addition of 50 ml of isopropanol, and cooling to 10 °C. The pH was adjusted to 2.0 by using 20 % isopropanol.HCl solution. After stirring for 1-2 hrs the product was filtered, washed with 20 ml of chilled isopropanol (10°C), followed by drying under vacuum to yield 7 g. The purity was more than 99.7 % by HPLC; melting range 213-214°C.

## Claims

1. Process for the preparation of a compound represented by formula (I) and salts thereof 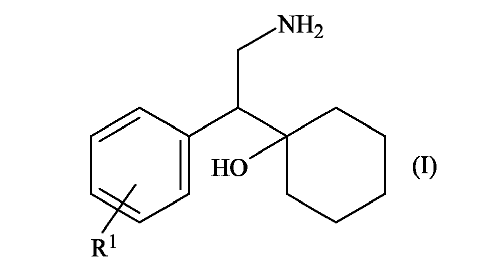 wherein R¹ represents H, OH, or an optionally substituted alkyl or alkoxyl group; said process comprising reacting a compound represented by formula (II) 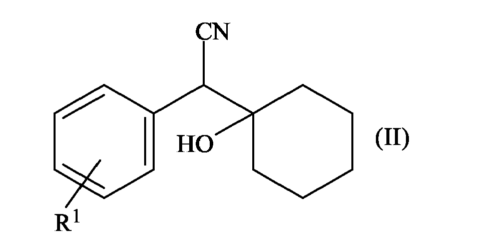 and a reducing borohydride agent selected from tetraalkylammonium borohydride and aralkyltrialkylammonium borohydride in the presence of an alkylhalide.

2. Process according to claim 1, wherein R¹ represents a para-methoxy group.

3. Process according to claim 1 or 2, further comprising passing hydrochloric acid gas through an organic solvent comprising the compound represented by formula (I) and isolating said compound as its hydrochloride salt by crystallization.

4. Process according to any one of the preceding claims wherein the reducing borohydride agent is tetrabutylammoniumborohydride.

5. Process according to any one of the preceding claims wherein the molar ratio of reducing borohydride agent and the compound represented by formula (II) is between 1:5 and 5:1.

6. Process according to any one of the preceding claims, wherein the compound represented by formula (II) is prepared by reacting an optionally substituted phenylacetonitrile and cyclohexanone in an aqueous hydroxide solution in the presence of a phase transfer catalyst.

7. Process according to claim 6, wherein the optionally substituted phenylacetonitrile and cyclohexanone are reacted at a temperature of between 10 and 25 °C.

8. Process according to claim 6 or 7, wherein the phase transfer catalyst is tetrabutylammonium bromide.

9. Process according to any one of claims 6-8, wherein the aqueous hydroxide solution is a sodium hydroxide solution.

10. Process for the preparation of a compound represented by formula (III) 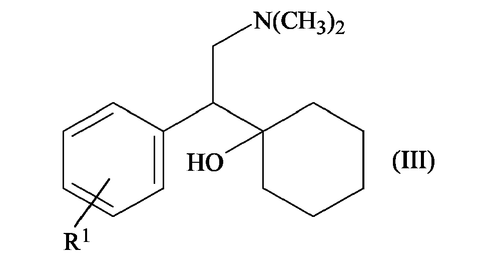 comprising the step of preparing the compound represented by formula (I) according to the process defined in any one of claims 1-9 and the subsequent step of methylating the compound represented by formula (I).

11. Process according to claim 10, wherein methylation is performed by heating to reflux a combination of formaldehyde, formic acid, water and the compound represented by formula (I).

12. Process for the preparation of a pharmaceutical preparation containing 0.01-25 wt% of the compound represented by formula (III), said process comprising the step of preparing said compound represented by formula (III) according to the process defined in claim 10 and subsequently combining said compound represented by formula (III) with one or more pharmaceutically acceptable excipients.
